# EUROPEAN PATENT APPLICATION

(11) **EP 3 069 746 A1**
(43) Date of publication of application: **21.09.2016**
(21) Application number: 14894572.8
(22) Date of filing: 12.06.2014
(51) Int. Cl.: A61M 16/06

(54) **HIGH-FLOW NASAL CANNULA**

(30) Priority: 11.06.2014 ES 201430818
(71) Applicant: Rodriguez Rodriguez, Gregorio, 28042 Madrid (ES)
(72) Inventor: Rodriguez Rodriguez, Gregorio, 28042 Madrid (ES)
(74) Representative: González López-Menchero, Álvaro Luis
(86) International application number: PCT/ES2014/070486
(87) International publication number: WO 2015/189438

(57) **Abstract**

The invention relates to a high-flow nasal cannula allowing an increased supply of oxygen flow compared to nasal cannulas that already exist on the market. To this end, the nasal cannula has a connection to an oxygen supply source from which a conduit extends, said conduit branching into two outflow conduits for supplying the nasal cannula, where the diameter of the outflow conduits leading from the branching point to the nasal cannula is such that it allows the guidance of the maximum flow supplied by the source. To this end, in one possible embodiment, the diameter of the supply tubes is at least equal to the diameter of the tube connecting to the source.

## Description

### OBJECT OF THE INVENTION

The object of the present invention, as described by the title of the invention, which is a high-flow nasal cannula, refers to a device used in oxygen therapy consisting of two small cannulas that are inserted into both nasal passages and have the unique feature of delivering a high and continuous flow of oxygen.

The present invention is characterized by the fact that it has a design that prevents a reduction in the flow supplied by the oxygen source and, therefore, enables a larger amount of oxygen to be delivered to the prongs of the nasal cannula.

Therefore, the present invention falls within the field of oxygen delivery devices and, specifically, nasal cannulas.

### BACKGROUND OF THE INVENTION

In the current state of the art, the nasal cannula is a well-known device which has a connection to an oxygen supply source from which a conduit extends and branches into two outflow conduits that extend to the nosepiece which has two perforated prongs that are inserted into each nasal passage and facilitate the delivery of oxygen.

Although these nasal cannulas perform the function they are designed for, there are aspects that could be improved, such as the flow of the oxygen supply in situations where high oxygen levels are required.

Due to the design of state-of-the-art nasal cannulas, the flow of oxygen delivered by the source may be choked and reduced as a consequence of the design of the Y-piece and the diameter of the tubes connecting to the nosepiece. In cases where the amount of oxygen needed ranges from 1 to 4 liters per minute, this reduction is not a problem. However, the nasal cannulas currently on the market are not able to deliver greater amounts of oxygen in cases where such amounts are required.

Therefore, the object of the present invention is to create a nasal cannula that delivers a greater amount of oxygen without being limited by its design to values from 1 to 4 liters per minute, developing a nasal cannula as described in the following section, the essential nature of which is established in the first claim.

### DESCRIPTION OF THE INVENTION

The object of the present invention relates to a high-flow nasal cannula, which allows for an increased supply of oxygen flow compared to nasal cannulas that already exist on the market. To this end, the nasal cannula has a connection to an oxygen supply source from which a conduit extends, said conduit branching into two outflow conduits for supplying the nasal cannula, where the diameter of the outflow conduits leading from the branching point to the nasal cannula is such that it allows the guidance of the maximum flow supplied by the source. To this end, in one possible embodiment, the diameter of the supply tubes is at least equal to the diameter of the tube connecting to the source.

Moreover, with the goal of preventing any reduction in the flow of oxygen at any point in the cannula, the prongs on the nosepiece have a diameter that is equal to the diameter of the supply tubes, or the tubes that extend from the Y-piece to the nosepiece.

Throughout the description and claims, the word "comprise" and its variants are not intended to exclude other technical characteristics, additives, components or steps. For those skilled in the art, other objects, advantages and characteristics of the invention may be deduced from both the description and the practical use of the invention.

### EXPLANATION OF THE DRAWINGS

In order to complement the description being made and with the object of helping to better understand the characteristics of the invention, in accordance with a preferred embodiment thereof, said description is accompanied, as an integral part thereof, by a set of drawings where, in an illustrative and non-limiting manner, the following has been represented:
In Figure 1, we can see a representation of a nasal cannula as in the current state of the art.

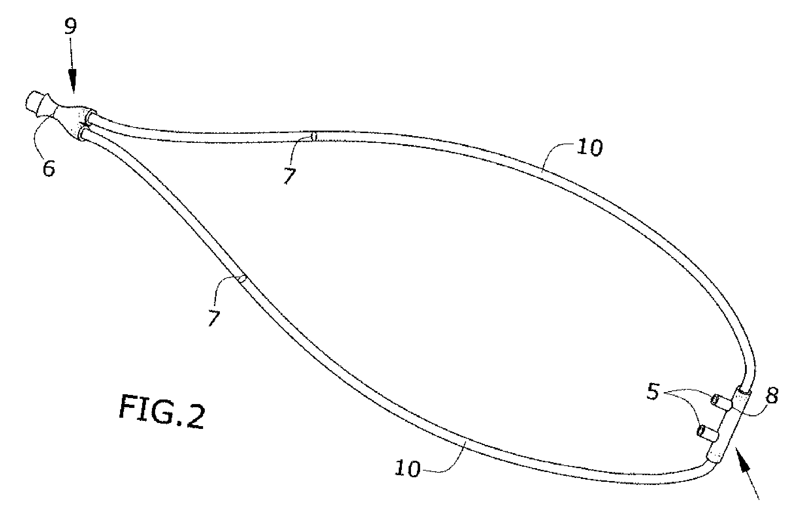

In Figure 2, we can see the design of the nasal cannula, object of the invention, which delivers a high flow of oxygen.

### PREFERRED EMBODIMENT OF THE INVENTION

With reference to the figures, below a preferred embodiment of the invention proposed is described.

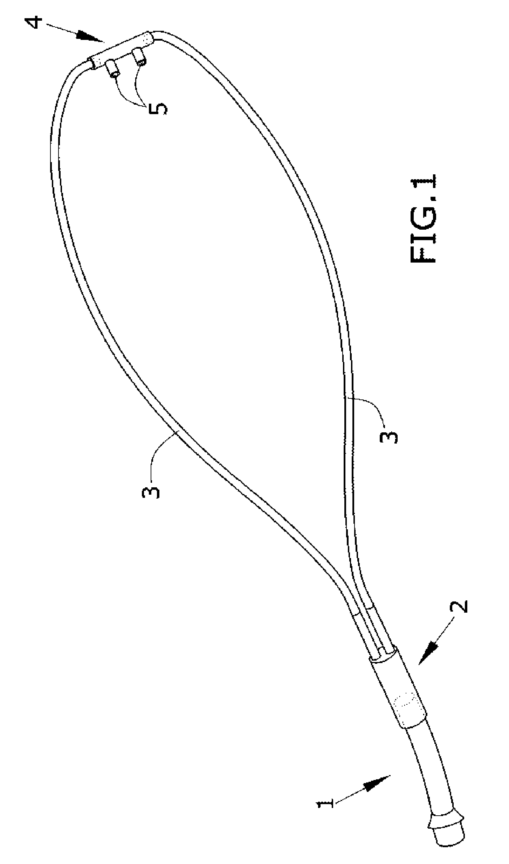

In figure 1, we can see that the state-of-the-art nasal cannula comprises a connection (1) to an oxygen supply source, to which a Y-piece (2) is connected, which in turn is connected to supply tubes (3) which supply oxygen to a nosepiece (4) with prongs (5) that are inserted into the nasal passages.

A unique characteristic shown in the figure is the fact that the supply tubes (3) have a smaller diameter than the diameter of the connection (1) to the source, which constricts the flow at the Y-piece (2) and, as a consequence, limits the amount of oxygen delivered.

To solve the aforementioned problems of a limited maximum flow found in state-of-the-art nasal cannulas, the design of the different parts has been modified in order to achieve greater flow, comprising, on the one hand, a Y-piece (9) and supply tubes (10) that extend from the Y-piece (9) to the nosepiece (4) in such a way that it does not cause any restriction in the flow supplied from the source.

To this end, in one possible embodiment, the diameter (7) of the supply tubes (10) is equal to or greater than the diameter (6) of the tube connecting to the source.

Moreover, the prongs (5) have a diameter (8) equal to the diameter (7) of the supply tubes (7), also with the objective of preventing a reduction in the oxygen flow delivered.

Thanks to the design of the nasal cannula which is the object of the invention, a high-flow nasal cannula with an improved level of blood oxygen saturation is achieved, avoiding the use of high-flow or oxygen delivery systems, such as oxygen masks or ventilators, and taking full advantage of the supply of the oxygen source.

Having thus adequately described the nature of the present invention, as well as how to put it into practice, it must be noted that, within its essential nature, the invention may be carried out according to other embodiments differing in detail from that set out by way of example, which the protection sought would equally cover, provided that the fundamental principle thereof is not altered, changed or modified.

## Claims

1. A high-flow nasal cannula **characterized in that** it comprises a Y-piece (9) and supply tubes (10) that extend from the Y-piece (9) to the nosepiece (4), such that it does not cause any reduction in the flow of oxygen from the source.

2. The high-flow nasal cannula according to claim 1, **characterized in that** the diameter (7) of the supply tubes (10) is equal to or greater than the diameter (6) of the tube connecting to the source.

3. The high-flow nasal cannula according to claim 2, **characterized in that** the nosepiece (4) has prongs (5) that have a diameter (8) that is equal to the diameter (7) of the supply tubes (7), also with the objective of preventing a reduction in the oxygen flow delivered.

## Amended claims

### Amended claims under Art. 19.1 PCT

1. A high-flow nasal cannula **characterized in that** it comprises a Y-piece (9) and supply tubes (10) that extend from the Y-piece (9) to the nosepiece (4), wherein the diameter (7) of the supply tubes (10) is equal to or greater than the diameter (6) of the tube connecting to the source, such that it does not cause any reduction in the flow supplied by the source.

2. The high-flow nasal cannula according to claim 1, **characterized in that** the nosepiece (4) has prongs (5) that have a diameter (8) that is equal to the diameter (7) of the supply tubes (7), also with the objective of preventing a reduction in the oxygen flow delivered.
